# EUROPEAN PATENT SPECIFICATION

(11) **EP 1 971 320 B1**
(45) Date of publication and mention of the grant of the patent: **06.07.2011**
(21) Application number: 06829737.3
(22) Date of filing: 15.12.2006
(51) Int. Cl.: A61K 8/899, A61Q 15/00

(54) **ANTIPERSPIRANT COMPOSITIONS**
ANTIPERSPIRANS-ZUSAMMENSETZUNGEN
FORMULES ANTITRANSPIRANTES

(30) Priority: 22.12.2005 GB 0526136; 05.12.2006 WO PCT/CN2006/003277
(43) Date of publication of application: 24.09.2008
(73) Proprietor: Unilever PLC, London EC4Y 0DY (GB); Unilever N.V., 3013 AL Rotterdam (NL)
(72) Inventor: COURTOIS, Jean-Philippe Andre Roger, Bebington, Wirral Merseyside CH63 3JW (GB); LIU, Weichang, Shanghai 200233 (CN); SMITH, Ian Karl, Bebington, Wirral Merseyside CH63 3JW (GB); WANG, Lin, Shanghai 200233 (CN); WHITE, Michael Stephen, Bebington, Wirral Merseyside CH63 3JW (GB); ZHANG, Qiqing, Shanghai 200233 (CN)
(74) Representative: Whaley, Christopher
(86) International application number: PCT/EP2006/012250
(87) International publication number: WO 2007/071375

(56) References cited:
- WO-A-00/25823
- WO-A-03/020771

## Description

### Field of Invention

This invention relates to the field of antiperspirant compositions and to methods of reducing perspiration. In particular, this invention is concerned with reducing perspiration on the surface of the human body by the use of water-soluble or water-dispersible thiomers.

### Background

Conventional antiperspirant actives are astringent metal salts, such as salts of aluminium and/or zirconium. Such materials can function extremely effectively; however, they can cause some problems, including the possibility of skin irritation.

Water-soluble or water-dispersible thiomers have not previously been recognised for their antiperspirant property, but they are well known in the field of drug delivery. They have been used, for example, in non-invasive polypeptide delivery and have been found to have mucoadhesive properties (Leitner et al, J. Pharm. Sci., 93(7), 1682, 2004). A wide range of such materials has been synthesised, including polyacrylic acid thiolated using cysteine (Bernkop-Schnurch et al, Drug Dev. Ind. Pharm., 30(1), 1, 2004). Such materials are also described in WO 00/25823 (Bernkop- Schnürch).

### Summary of the Invention

We have discovered that water-soluble or water-dispersible thiomers may be used as antiperspirant actives and may be formulated in effective antiperspirant compositions.

In a first aspect of the invention, there is provided an antiperspirant composition as defined in claim 1 comprising a carrier substance and a water-soluble or water-dispersible thiomer which is a thiolation product of polyethyleneimine.

In a second aspect of the invention, there is provided a method of manufacture of an antiperspirant composition, comprising the dispersion of a thiomer which is a thiolation product of polyethyleneimine in a carrier substance.

In a third aspect of the invention, there is provided a method of reducing perspiration comprising the delivery to the surface of the human body of a composition comprising a water-soluble or water-dispersible thiomer which is a thiolation product of polyethyleneimine.

In a fourth aspect of the invention, there is provided the use of a water-soluble or water-dispersible thiomer which is a thiolation product of polyethyleneimine as an antiperspirant active.

### Detailed Description

The compositions of the invention may take any of the product forms known in the art. Thus, they may be sticks, soft solids, gels, aerosols, or liquids (for roll-on application or other means of application). All of the compositions of invention comprise a carrier substance and a water-soluble or water-dispersible thiomer; in general, they comprise an anhydrous continuous phase with the water-soluble thiomer dispersed therein.

It is preferred that compositions according to invention are anhydrous. Throughout this specification, the term "anhydrous" should be understood to mean having less than 10% by weight of water, in particular less than 5% by weight of water, and especially less than 1% by weight of water.

The compositions of the invention may be applied to the surface of the human body to deliver an antiperspirancy benefit. They are particularly suitable for application to the underarm regions of the human body.

### Thiomers

Thiomers are thiolated polymers. Preferred thiomers are polymers having from 0.5% to 20% of their repeat units comprising a thiol group. These thiol groups may be introduced by copolymerisation or by thiolation of a pre-formed base polymer (*vide infra*).

Throughout this specification, the term 'polymer' should be understood to include co-polymers comprising more than one type of repeat unit.

Without wishing to be bound by theory, it is believed that the thiol groups of the thiomer enable or enhance the polymer's ability to act as a mucoadhesive and that this ability enables or enhances the antiperspirant activity of the thiomer. "Mucoadhesives" are materials that can attach to mucin in a biological surface. It is believed that the antiperspirant activity results, at least in part, from the ability of the thiomers to act as pore blockers. The thiomers, when swollen by water, are believed to serve to as plugs that may, at least in part, block the exit of sweat from eccrine sweat glands.

It is essential that the thiomer is water-soluble or water-dispersible in order for it to dissolve or disperse in eccrine sweat. Water-dispersible thiomers should be understood to be thiomers that are dispersible in water to a sufficient extent for them to function as mucoadhesive, self-gelling polymers in the manner described herein. Preferred thiomers are water-soluble, having a water-solubility of at least 1 g.dm⁻³, in particular at least 10 g.dm⁻³, and especially at least 100 g.dm⁻³.

Once the thiomer has dissolved or dispersed in the eccrine sweat, it is able to start acting as an antiperspirant active. In order to do this to the best advantage, it is highly desirable that the thiomer is self-gelling when dissolved in water at 37°C. When dissolved or dispersed in water, such a self-gelling thiomer causes the viscosity of the solution of which it is a part to increase with time. The most effective thiomers, when dissolved or dispersed in water at a concentration of 100 g.dm⁻³, will increase the viscosity of the water by at least 100% within 30 minutes of being dissolved at 37°C.

More than one thiomer may be used in compositions of the invention. The total amount of thiomer incorporated into the compositions of the invention is preferably from 0.01% to 50%, more preferably from 0.1% to 25%, and most preferably from 1% to 25% by weight of the total composition.

In one embodiment of the invention, the thiomer is present in the form of a particulate dispersion within the carrier substance (*vide infra*), the carrier substance typically comprising an anhydrous continuous phase, as defined above. For reasons of composition stability and/or efficacy, the particulate dispersion of thiomer preferably has at least 90% of the particulates having a particle size of from 1 to 100 microns.

In a second embodiment of the invention, the thiomer is dissolved in one or more of the components of the carrier substance. This embodiment offers benefits of compositional stability and/or efficacy, benefits that may be further enhanced when the composition is homogenous. The carrier substance typically comprises an anhydrous continuous phase with the thiomer dissolved in this or in a liquid phase dispersed therein. When the thiomer is dissolved in a liquid phase dispersed in an anhydrous continuous phase, the emulsion is preferably anhydrous, the liquid disperse phase preferably comprising a C₁-C₆ mono- or polyhydric alcohol.

The water-soluble or water-dispersible thiomers have as their non-thiolated base polymer a polymer that is itself water-soluble or water-dispersible and which is a polyethyleneimine. The non-thiolated base polymer of a thiomer should be considered as the thiomer minus the monomer units possessing thiol groups, if said groups were introduced by copolymerisation, and as the polymer prior to thiolation, if thiolation of a pre-formed polymer was employed. Preferably, the non-thiolated base polymer is water soluble, having a water solubility of at least 1 g.dm⁻³, in particular at least 10 g.dm⁻³, and especially at least 100 g.dm⁻³. It is highly preferred that the base polymer is mucoadhesive, for example a polymer of vinylpyrrolidone.

For optimum performance, the molecular weight of the thiomer is preferably from 1 Da to 5,000 kDa, more preferably from 5 kDa to 2,000 kDa, and most preferably from 20 kDa to 1,000 kDa.

Introduction of the thiol groups of the thiomer may be by any of the means known in the art; co-polymerisation with a source of a thiol group is one such means. This may be done by free radical co-polymerisation.

The source of the thiol groups may be N,N'-bisacryloylcystamine, the disuphide bonds later being broken to give free thiol groups, or an N,N-dialkyldithiocabamate (e.g. vinylbenzyl-N,N-diethyldithiocarbamate, the thiocarbamate later being hydrolysed to release the free thiol groups.

Introduction of the thiol groups of the thiomer may be done by thiolation of a pre-formed base polymer. A variety of techniques may be employed to do this:
i). Polymers comprising carboxylic acid groups may be linked to cysteine using known amidation procedures;
ii). Polymers comprising amine groups (e.g. chitosan) may be:
   a) Linked to a thiol-containing acid such as cysteine or thioglycolic acid; or
   b) Thiolated through the use of a heterobifunctional reagent such as N-succinimdyl-3-(pyridyldithio)propionate; or
   c) Thiolated using 2-iminothiolane (Traut's reagent);
iii). Polymers comprising hydroxyl groups may be:
   a) Derivatised with 2-iminothiolane (Traut's reagent); or
   b) Activated by reaction with a chloroformate active ester (e.g. 4-nitrophenylformate) and then reacted with a bi-functional thiol such as 2-aminothioethanol.

Thiomers that are the product of thiolation of a pre-formed base polymer are preferred, in particular thiomers that are produced via thiolation using cysteine. The thiomers are derived from polyethyleneimine. Especially preferred thiomers of this type have at least some of the primary amine groups of the original PEI converted to thiolated amide groups of formula:

R-NH-CO.(CH₂)ₙ-SH

where R represents the remainder of the polymer and n is from 2 to 5, preferably from 3 to 4, and most preferably 3. The extent of conversion is such that preferably from 1 to 50%, more preferably from 5 to 50%, and most preferably from 25-50% of the primary amine groups are converted.

### Carrier Substances

The carrier substance may be any substance in which the thiomer may be dissolved or otherwise dispersed. In order to attain an effective composition, it is highly desirable that the carrier substance is chemically compatible with the thiomer. It is also highly desirable that the carrier substance is cosmetically acceptable, so that the composition may be used in cosmetic application.

The carrier substance may comprise more than one component and the term 'carrier substance' should be understood to encompass all of the components of the compositions of the invention except thiomer. Therefore, it is highly desirable that all of the 'non-thiomer' components of the composition are chemically compatible with the thiomer and that they are all cosmetically acceptable, for the reasons stated in the above paragraph.

The choice of carrier substance will depend upon the product form and properties desired. It is preferred that the carrier substance comprises less 20% by weight of water and it is more preferred that the carrier substance is anhydrous, as previously defined.

In stick and soft solid products, it generally required to incorporate a structurant. In general, the amount of structurant will be from 0.1 to 25% and particularly from 1 to 15% by weight. Often such structurants are used to thicken or structure an anhydrous continuous phase having the thiomer dispersed therein. Suitable structurants for this latter purpose are described hereinbelow.

A suitable structurant may be an organic polymer which is soluble in the anhydrous continuous phase, though commonly at an elevated temperature of above 60°C. Such polymers are particularly well suited to producing compositions in the form of soft or firm solids. Such polymers can be selected from polysaccharides esterified with a fatty acid, e.g. dextrin palmitate; polyamides, as discussed in US 5;500,209, e.g. Versamid 950™; alkylene/arylene block copolymers, e.g. ethylene/styrene, propylene and/or butylene block co-polymers such SEBS block copolymers; alkyl substituted galactomannan, e.g. N-HANCE™; or co-polymers of vinyl(pyrrolidone) and ethylene containing at least 25 methylene units. The concentration of such polymers in the anhydrous continuous phase is generally from 1 to 20%.

A suitable structurant may be a wax. Suitable waxes include beeswax, candelilla wax, carnauba wax, and other waxes having similar properties. Such other waxes include hydrocarbon waxes, e.g. paraffin wax, mineral wax and microcrystalline wax; synthetic waxes, such as polyethylene of 2000 to 10000 daltons; waxy derivatives or waxy components of natural waxes, such as ester components, either extracted or synthesised, solid ester derivatives of glycerol or glycol, typically with linear saturated fatty acids, usually containing a significant fraction of C₁₆₋₂₂ acid residues, which may be synthesised or obtained by hydrogenating the corresponding natural oil; petroleum waxes, waxy silicone polymers containing alkyl substituents of at least C₁₀ chain length; and waxy fatty alcohols, that normally are linear and often contain from 14 to 24 carbons, such as stearyl alcohol, cetyl alcohol and/or behenyl alcohol.

A suitable structurant may be an oil-soluble polyamide or amide/silicone copolymer; an hydroxystearic acid, e.g. 12-hydroxystearic acid, or ester or amide derivatives thereof; an N-acylaminoacid amide and ester as described in US 3,969,087, such as, in particular, N-Lauroyl-L-glutamic acid di-n-butylamide; an amide derivative as set forth in WO 98/27954, notably an alkyl N,N'dialkyl succinamides; threitol or like amido gellants as set forth in US-A-6410001; lanosterol, as set forth in US 6,251,377; an amido derivative of cyclohexane, as set forth in US 6,410,003; a combination of a sterol and a sterol ester, as set forth in WO 00/61096, e.g. γ-oryzanol and β-sitosterol; or a fatty acid ester of cellobiose, e.g. cellobiose octanonanoate comprising a minor fraction of cellobiose heptanonanoate.

In aerosol products, it is generally required to incorporate a volatile propellant or a compressed gas. When a volatile propellant is employed, it is typically present at from 30% to 99% and particularly at from 3-5% to 87% by weight of the total composition. The volatile propellant may be selected from liquefied hydrocarbons or halogenated hydrocarbon gases (particularly fluorinated hydrocarbons such as 1,1-difluoroethane and/or 1-trifluoro-2-fluoroethane) that have a boiling point of below 10°C and especially those with a boiling point below 0°C. It is preferred to employ non-chlorinated volatile propellants. It is especially preferred to employ liquefied hydrocarbon gases, and especially C₃ to C₆ hydrocarbons, including propane, isopropane, butane, isobutane, pentane and isopentane and mixtures of two or more thereof. Preferred propellants are isobutane, isobutane/isopropane, isobutane/propane and mixtures of isopropane, isobutane and butane. In some embodiments, dimethylether may be employed as a volatile propellant.

When a gas compressed is employed, it is typically a nonreactive gas such air, nitrogen or carbon dioxide.

In liquid products, it is required to incorporate a liquid carrier substance. A preferred product type is a particulate dispersion of the thiomer in the liquid carrier substance. The liquid carrier substance may be selected from the liquid materials described as sensory modifiers and described hereinafter. A preferred liquid carrier substance is a volatile silicone, because such a liquid can give a drier feel to the applied film after the composition is applied to skin. The carrier substance may contain from 0.1 to 50% and particularly from 10 to 40% by weight volatile silicone. To class as "volatile" such material should have a measurable vapour pressure at 20 or 25°C. Typically the vapour pressure of a volatile silicone lies in a range from 1 or 10 Pa to 2 kPa at 25°C. A volatile silicone may also be included in a stick, soft solid, or aerosol product.

Volatile silicones can be linear or cyclic or mixtures thereof. Preferred cyclic silicones include polydimethylsiloxanes and particularly those containing from 3 to 9 silicon atoms and preferably not more than 7 silicon atoms and most preferably from 4 to 6 silicon atoms, otherwise often referred to as cyclomethicones. Preferred linear siloxanes include polydimethylsiloxanes containing from 3 to 9 silicon atoms. The volatile siloxanes normally by themselves exhibit viscosities of below 10⁻⁵ M²/sec (10 centistokes), and particularly above 10⁻⁷ M²/sec (0.1 centistokes), the linear siloxanes normally exhibiting a viscosity of below 5 x 10⁻⁶ m²/sec (5 centistokes). The volatile silicones can also comprise branched linear or cyclic siloxanes such as the aforementioned linear or cyclic siloxanes substituted by one or more pendant -0-Si(CH₃)₃ groups. Examples of commercially available silicone oils include oils having grade designations 344, 345, 244, 245 and 246 from Dow Corning Corporation; Silicone 7207 and Silicone 7158 from Union Carbide Corporation; and SF1202 from General Electric.

Thickeners are preferred optional components in liquid products. Such materials include particulate inorganic substances, such as clays or finely divided silica. Such materials are also preferred optional components in aerosol products, in which they are often referred to as suspending agents. Propylene carbonate may also be used in aerosol products to serves as a suspending agent.

In all product forms, it may be desirable to incorporate a conventional antiperspirant active to augment the antiperspirancy of the thiomer and to act as an anti-microbial active and deodorant. A conventional antiperspirant active should be understood to be an astringent metal salt, such as a salt of aluminium and/or zirconium. Examples of suitable conventional antiperspirant salts include aluminium, zirconium and aluminium/zirconium halides and halohydrate salts, such as chlorohydrates. When included, preferred levels of incorporation are from 0.1% to 50%, particularly from 1% to 25% and especially from 5% to 15% by weight of the composition. Especially preferred aluminium halohydrate salts, known as activated aluminium chlorohydrates, are described in EP 6,739 (Unilever PLC and NV). Zirconium aluminium chlorohydrate actives are also preferred materials, as are the so-called ZAG (zirconium-aluminium-glycine) complexes, for example those disclosed in US 3,792,068 (Procter and Gamble Co.). Zinc phenol sulphonate may also be used, preferably at up to 3% by weight of the composition.

In all product forms, and particularly those comprising a conventional antiperspirant salt, it may be desirable to incorporate a sensory modifier to improve skin feel. Emollients, humectants, volatile oils and non-volatile oils are all suitable classes of sensory modifiers. Examples of such materials include cyclomethicone, dimethicone, dimethiconol, isopropyl myristate, isopropyl palmitate, C12-C15 alcohol benzoate, PPG-3 myristyl ether, octyl dodecanol, isostearyl alcohol, C7-C14 isoparaffins, di-isopropyl adipate, isosorbide laurate, PPG-14 butyl ether, glycerol, hydrogenated polyisobutene, polydecene, phenyl trimethicone, dioctyl adipate, and hexamethyl disiloxane. When employed, the total amount of sensory modifier is preferably from 0.5% to 50%, more preferably from 1% to 30%, and most preferably from 3% to 20% by weight of the total composition.

In all product forms, and particularly those lacking a conventional antiperspirant salt, it is highly desirable to incorporate an organic anti-microbial agent to act as a preservative and/or as a deodorant when the product is applied to the surface of the human body. Most of the classes of agents commonly used in the art can be incorporated into compositions of the invention. Levels of incorporation are preferably from 0.01% to 3% and more preferably from 0.03% to 1% by weight of the total composition. Preferred compositions of the invention comprise an organic anti-microbial agent having a minimum inhibitory concentration (MIC) of 1 mg.ml⁻¹ or less, particularly 200 µg.ml⁻¹ or less, and especially 100 µg.ml⁻¹ or less. The MIC of an anti-microbial agent is the minimum concentration of the agent required to significantly inhibit microbial growth. Inhibition is considered "significant" if an 80% or greater reduction in the growth of an inoculum of a relevant micro- organism is observed, relative to a control medium without an anti-microbial agent, over a period of 16 to 24 hours at 37°C. The "relevant microorganism" used for testing should be representative of those associated with the substrate to be treated. When the substrate to be treated is human skin, a relevant microorganism is *Staphylococcus epidermidis.* Details of suitable methods for determining MICs can be found in "Antimicrobial Agents and Susceptibility Testing", C.Thornsberry, (in "Manual of Clinical Microbiology", 5th Edition, Ed. A. Balows et al, American Society for Microbiology, Washington D.C., 1991). A particularly suitable method is the Macrobroth

Dilution Method as described in Chapter 110 of above publication (pp. 1101-1111) by D. F. Sahm and J. A. Washington II. MICs of a organic anti-microbials suitable for inclusion in the composition of the invention are triclosan: 0.01-10 µg.ml⁻¹ (J.Regos et al., Dermatologica (1979), 158: 72-79) and farnesol: ca. 25 µg.ml⁻¹ (K. Sawano, T. Sato, and R. Hattori, Proceedings of the 17^{th} IFSCC International Conference, Yokahama (1992) p.210-232). By contrast ethanol and similar alkanols have MICs of greater than 1 mg.ml⁻¹. Preferred organic anti-microbials are bactericides, for example quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred organic anti-microbials for use in the compositions of the invention are polyhexamethylene biguanide salts (also known as polyaminopropyl biguanide salts), an example being Cosmocil CQ™ available from Zeneca PLC, preferably used at up to 1% and more preferably at 0.03% to 0.3% by weight; 2',4,4'-trichloro,2-hydroxy-diphenyl ether (triclosan), preferably used at up to 1% by weight of the composition and more preferably at 0.05-0.3%; and 3,7,11-trimethyldodeca-2,6,10-trienol (farnesol), preferably used at up to 1% and more preferably at up to 0.5% by weight of the composition. Organic anti-microbials that are iron (III) chelators may be employed, either alone or in combination with another organic anti-microbial agent. Chelators having an iron (III) binding constant of 10²⁶ or greater are preferred, with chelators having an iron (III) binding constant of 10²⁸ or greater being particularly preferred. Especially preferred iron (III) chelators are N,N'-ethylenebis[2-(2-hydroxyphenyl)glycine] (EDDHA), triethylenetetraaminehexaacetic acid (TTHA), and diethylenetriaminepentaacetic acid (DTPA).

Iron (III) chelators are, in general, acids. They may be used as such in the compositions of the invention, although in compositions having an anhydrous continuous phase they are preferably used as a salt or acid salt of a protonated or quaternised amine. Preferred amines for neutralisation of the chelator are 2-amino-2-methyl-1-propanol, diisopropanolamine, and 2-aminobutan-1-ol, cyclohexylamine.

Other anti-microbial agents that may be used in compositions of the invention, in particular those also comprising an iron (III) chelator, are transferring dissociation promoters such as BHT (butylated hydroxytoluene), as described in US 6,503,490 (Unilever, 2003).

In all product forms, it is highly desirable to incorporate a perfume. Suitable materials include conventional perfumes, such as perfume oils and also include so-called deo-perfumes, as described in EP 545,556 and other publications. Levels of incorporation are preferably up to 4% by weight, particularly from 0.1% to 2% by weight, and especially from 0.7% to 1.7% by weight.

In all product forms, it may be desirable to include one or more wash-off aids, often in a proportion of up to about 10% by weight, especially up to about 5% by weight and particularly from 0.5 to 3% by weight. Such wash off aids commonly comprise nonionic surfactants and especially nonionic surfactants which contain a polyalkylene oxide moiety, the residue of a fatty acid or fatty alcohol, and optionally the residue of an aliphatic polyhydric alcohol linking group. Although, the surfactants may comprise a single fatty residue, they preferably contain two residues. Preferably, the surfactant is an ester surfactant, and especially a diester surfactant. The polyalkylene oxide is often polyethylene oxide, or polypropylene oxide or mixed polyethylene oxide/propylene oxide, the polymer containing from 3 to 50 and especially from 5 to 20 alkylene oxide units. The fatty residue often derives from a fatty acid or alcohol containing from 12 to 24 carbons, which in many instances is linear, examples including 16, 18 or 22 linear carbons. Especially preferred wash-off aids herein comprise polyethylene oxide diesters of fatty alcohols containing 16 to 22 linear carbons, such as PEG-8 distearate.

### Methods of Manufacture

The compositions of the invention may be manufactured by any means that allows the dispersion of a particulate thiomer in an anhydrous continuous phase. In a first embodiment, a particulate thiomer is dispersed an anhydrous continuous phase.

In a second embodiment, a thiomer is dissolved in one or more of the components of the carrier substance. This may involve dissolving the thiomer in an anhydrous continuous phase. Alternatively, it might involve dissolving the thiomer in a suitable solvent and then dispersing the thiomer solution so formed in an anhydrous continuous phase. A suitable solvent is one that is able to dissolve the thiomer.

### Examples

The following non-limiting examples may be prepared and used in accordance with the invention. The thiomers employed in the formulation examples may be selected from those in the preceding description. Amounts indicated are percentages by weight of the total composition, unless otherwise stated.

### Examples 6 to 17: Preparation of Thiolated Polyethyleneimine (PEI)

PEI polymer samples of molecular weight 25, 70-90, and 750 kDa (Lupasol P) were thiolated using γ-thiobutyrolactone, using methods known in the art (See, for example, T. Takagishi et al, Biopolymers, 1972, 11, 483). The products indicated in Table 2 were obtained.

**Table 2: Thiolated PEIs**

| | **Example** | | |
|---|---|---|---|
| PEI MW: | 25k | 70-90k | 750k |
| Thiolation: | | | |
| 25% | 6 | 7 | 8 |
| 50% | 9 | 10 | 11 |
| 75% | 12 | 13 | 14 |
| 100% | 15 | 16 | 17 |

The thiolation percentages given refer to the theoretical extent to which the primary amine groups of the original PEI are converted to thiolated amide groups, based upon the amount of γ-thiobutyrolactone used in the reaction. The structure of the thiolated amide groups may be represented as -NH-CO.CH₂CH₂CH₂SH.

Aqueous solutions of each of Examples 6 to 17 were prepared at 10% by weight and pH 7. Unlike analogous solutions of the corresponding PEI base polymers, these thiomer solutions were found to form gels within 5 minutes (in the presence of excess hydrogen peroxide - used to accelerate oxidative crosslinking of the thiol groups). This is an indication of the expected improvement in self-gelling with the thiolated polymers.

**Table 4: Stick Formulations**

| **Components** | **Examples** | | |
|---|---|---|---|
| | **24** | **25** | **26** |
| Thiomer | 20 | 20 | 10 |
| AZAG (1) | - | - | 10 |
| Suprafino talc | 3.2 | 3.2 | 3.2 |
| Stearyl alcohol | 14.0 | 14.0 | 14.0 |
| Hydrogenated castor oil | 4.0 | 4.0 | 4.0 |
| PEG-8 distearate | 1.0 | 1.0 | 1.0 |
| PHMBS (2) | - | 0.1 | - |
| DC245 (3) | To 100 | To 100 | To 100 |

1. Activated aluminium-zirconium-glycine complex.
2. Poly(hexamethylenebiguanide) stearate, ex Arch chemicals.
3. Volatile silicone, ex Dow Corning.

**Table 5: Aerosol Formulations**

| **Components** | **Examples** | | |
|---|---|---|---|
| | **27** | **28** | **29** |
| Thiomer | 3 | 5 | 2.5 |
| AACH (1) | - | - | 2.5 |
| DC245 (2) | 7.3 | 7.3 | 7.3 |
| Bentone 38V (3) | 0.5 | 0.5 | 0.5 |
| Propylene carbonate | 0.2 | 0.2 | 0.2 |
| PHMBS (4) | - | 0.1 | - |
| CAP40 (5) | To 100 | To 100 | To 100 |

1. Activated aluminium chlorohydrate.
2. Volatile silicone, ex Dow Corning.
3. Quaternium-18-hectorite, ex Rheox.
4. Poly(hexamethylenebiguanide) stearate, ex Arch chemicals.
5. Volatile propellant, proprietary mix of butane, isobutane and propane, Ex. Calor.

## Claims

1. An antiperspirant composition comprising a carrier substance and a water-soluble or water-dispersible thiomer and having an anhydrous continuous phase with the thiomer dispersed therein, **characterised in that** the thiomer is thiolation product of polyethyleneimine.

2. An antiperspirant composition according to claim 1, wherein the thiomer is a mucoadhesive.

3. An antiperspirant composition according to claim 1 or claim 2, wherein the thiomer is self-gelling when dissolved in water at 37°C.

4. An antiperspirant composition according to any of the preceding claims, wherein the thiomer is water-soluble, having a water-solubility of at least 1 g.dm⁻³.

5. An antiperspirant composition according to any of the preceding claims, comprising a conventional antiperspirant active.

6. An antiperspirant composition according to any of the preceding claims, comprising a sensory modifier.

7. An antiperspirant composition according to any of the preceding claims, comprising an organic anti-microbial agent.

8. A method of manufacture of an antiperspirant composition, comprising the dispersion of a thiomer in a carrier substance having an anhydrous continuous phase, **characterised in that** the thiomer is thiolation product of polyethyleneimine.

9. A non-Therapeutic method of reducing perspiration comprising the delivery to the surface of the human body of a composition comprising a water-soluble or water-dispersible thiomer, **characterised in that** the thiomer is thiolation product of polyethyleneimine.

10. The use of water-soluble or water-dispersible thiomer as an antiperspirant active, **characterised in that** the thiomer is thiolation product of polyethyleneimine.

## Patentansprüche

1. Schweißhemmende Zusammensetzung, die eine Trägersubstanz und ein wasserlösliches oder wasserdispergierbares Thiomer umfasst und eine wasserfreie kontinuierliche Phase mit dem Thiomer darin dispergiert hat, **dadurch gekennzeichnet, dass** das Thiomer ein Thiolierungsprodukt von Polyethylenimin ist.

2. Schweißhemmende Zusammensetzung gemäß Anspruch 1, wobei das Thiomer mucoadhäsiv ist.

3. Schweißhemmende Zusammensetzung gemäß Anspruch 1 oder Anspruch 2, wobei das Thiomer selbstgelierend ist, wenn es in Wasser mit 38 °C gelöst wird.

4. Schweißhemmende Zusammensetzung gemäß einem der vorangehenden Ansprüche, wobei das Thiomer wasserlöslich ist, eine Wasserlöslichkeit von wenigstens 1 g.dm⁻³ hat.

5. Schweißhemmende Zusammensetzung gemäß einem der vorangehenden Ansprüche, die einen herkömmlichen schweißhemmenden Wirkstoff umfasst.

6. Schweißhemmende Zusammensetzung gemäß einem der vorangehenden Ansprüche, die ein sensorisches Modifizierungsmittel umfasst.

7. Schweißhemmende Zusammensetzung gemäß einem der vorangehenden Ansprüche, die ein organisches antimikrobielles Mittel umfasst.

8. Verfahren zur Herstellung einer schweißhemmenden Zusammensetzung, umfassend die Dispersion eines Thiomers in einer Trägersubstanz, die eine wasserfreie kontinuierliche Phase hat, **dadurch gekennzeichnet, dass** das Thiomer ein Thiolierungsprodukt von Polyethylenimin ist.

9. Nicht-therapeutisches Verfahren zur Reduzierung der Perspiration, umfassend die Abgabe einer Zusammensetzung, die ein wasserlösliches oder wasserdispergierbares Thiomer umfasst, an die Oberfläche des menschlichen Körpers, **dadurch gekennzeichnet, dass** das Thiomer ein Thiolierungsprodukt von Polyethylenimin ist.

10. Verwendung eines wasserlöslichen oder wasserdispergierbaren Thiomers als schweißhemmenden Wirkstoff, **dadurch gekennzeichnet, dass** das Thiomer ein Thiolierungsprodukt von Polyethylenimin ist.

## Revendications

1. Composition antitranspirante comprenant un excipient et un thiomère hydrosoluble ou hydrodispersible et ayant une phase continue anhydre dans laquelle est dispersé le thiomère, **caractérisée en ce que** le thiomère est un produit de thiolation de la polyéthylèneimine.

2. Composition antitranspirante selon la revendication 1, dans laquelle le thiomère est une substance mucoadhésive.

3. Composition antitranspirante selon la revendication 1 ou la revendication 2, dans laquelle le thiomère est auto-gélifiant lorsqu'il est dissous dans l'eau à 37 °C.

4. Composition antitranspirante selon l'une quelconque des revendications précédentes, dans laquelle le thiomère est hydrosoluble et présente une hydrosolubilité d'au moins 1 g.dm⁻³.

5. Composition antitranspirante selon l'une quelconque des revendications précédentes, comprenant un actif antitranspirant conventionnel.

6. Composition antitranspirante selon l'une quelconque des revendications précédentes, comprenant un modificateur sensoriel.

7. Composition antitranspirante selon l'une quelconque des revendications précédentes, comprenant un agent antimicrobien organique.

8. Méthode de préparation d'une composition antitranspirante, comprenant la dispersion d'un thiomère dans un excipient ayant une phase continue anhydre, **caractérisée en ce que** le thiomère est un produit de thiolation de la polyéthylèneimine.

9. Méthode non thérapeutique pour réduire la transpiration comprenant l'application à la surface du corps humain d'une composition comprenant un thiomère hydrosoluble ou hydrodispersible, **caractérisée en ce que** le thiomère est un produit de thiolation de la polyéthylèneimine.

10. Utilisation d'un thiomère hydrosoluble ou hydrodispersible en tant qu'actif antitranspirant, **caractérisée en ce que** le thiomère est un produit de thiolation de la polyéthylèneimine.
